# EUROPEAN PATENT APPLICATION

(11) **EP 2 859 841 A1**
(43) Date of publication of application: **15.04.2015**
(21) Application number: 14188287.8
(22) Date of filing: 09.10.2014
(51) Int. Cl.: A61B 5/00, G04G 21/02, A61B 5/024

(54) **Biological information measurement device**

(30) Priority: 11.10.2013 JP 2013213480
(71) Applicant: Seiko Epson Corporation, Shinjuku-ku Tokyo (JP)
(72) Inventor: Takei, Yukio, Nagano, 392-8502 (JP); Mochimaru, Takeshi, Nagano, 392-8502 (JP); Kamijo, Takahiro, Nagano, 392-8502 (JP)
(74) Representative: Hoffmann Eitle

(57) **Abstract**

A biological information measurement device includes: a sensor section (280) which is configured to detect biological information of a test object; a processing section (240) which is configured to perform processing of the biological information; a power supply section (260) which is configured to supply electric power to the sensor section (240) and the processing section (260); a device main body having a case section (200) in which the sensor section (280), the processing section (240), and the power supply section (260) are accommodated; and a band section (34,44) for mounting the device main body on the test object, wherein the weight of the device main body is less than or equal to 60 g, and it is possible to measure biological information continuously for 10 hours or more.

## Description

### BACKGROUND

### 1. Technical Field

The present invention relates to a biological information measurement device which measures the biological information of a test object.

### 2. Related Art

In the past, an electronic device such as a measurement device which is mounted on the wrist or the like of a wearer and measures biological information such as the pulse or a wristwatch having a function of measuring the biological information has been known. In such an electronic device, it is necessary to bring a device main body which performs measurement into close contact with an appropriate site such as a wrist of the wearer.

A biological information measurement device described in JP-A-2012-90975 is provided with first and second band members mounted on a device main body, and a connecting member. Each band member has a stretchable portion which expands and contracts along a band extension direction, and the stretchable portion has flexibility and has a first slit having a pair of slits following a width direction and a slit following the band extension direction, and a pair of second slits communicating with the outside of the stretchable portion along the width direction. The connecting member is provided with a fixed member, a sliding member slidable along the band extension direction of the first band member, and a biasing member which biases the sliding member in the opposite direction to the band extension direction, and the sliding member is provided with a connection portion (a projecting bar) which is connected to the second band member. Further, in such a biological information measurement device, a power supply which drives a sensor for performing the measurement of biological information or an electronic circuit is installed. In JP-A-2012-90975, a configuration is disclosed in which as a power supply section, a rechargeable battery is provided and the battery is installed in the device main body which is mounted on a test object.

However, in the biological information measurement device described in JP-A-2012-90975 described above, if the frequency of detecting biological information increases, battery drain is promoted, and power is further consumed in order to use a display section having a large screen, and therefore, there is a problem in that measurement time is shortened. Further, if the capacity of the battery is increased, although it is possible to make the measurement time be a long period of time, the weight and the size of the outer shape of the device main body increase, and thus there is also a problem in that a feeling of fatigue due to mounting is given to a test object. Further, in a case where the weight of a device main body section of a biological information measurement device is large, there is a concern that it may adversely affect biological information measurement at the time of motion. Then, in a case where the thickness or the volume of the device main body section of the biological information measurement device is larger than necessary, since the device main body is greatly visible, a visual feeling of pressure is given to a test object, and therefore, there is also a problem in that it is difficult to measure biological information continuously for a long period of time.

### SUMMARY

An advantage of some aspects of the invention is to solve at least a part of the problems described above, and the invention can be implemented as the following forms or application examples.

### Application Example 1

This application example of the invention is directed to a biological information measurement device including: a sensor section which detects biological information of a test object; a processing section which performs processing of the biological information; a power supply section which supplies electric power to the sensor section and the processing section; a device main body having a case section in which the sensor section, the processing section, and the power supply section are accommodated; and a band for mounting the device main body on the test object, wherein weight of the device main body is less than or equal to 60 g, and it is possible to measure biological information continuously for 10 hours or more.

According to such a biological information measurement device, it is possible to measure the biological information of a test object by the sensor section provided in the device main body mounted on the test object by the band. Further, the biological information measurement device is provided with the power supply section in which it is possible to supply a power supply capable of measuring biological information continuously for 10 hours or more, and the weight of the device main body is less than or equal to 60 g. Therefore, an inertial force due to the motion of a test object is suppressed from acting on the test object and the device main body, and thus it is possible to measure biological information with high precision continuously for 10 hours or more. Further, it is possible to realize a biological information measurement device in which, even if the biological information measurement device is mounted for a long period of time, a feeling of pressure or a feeling of fatigue is suppressed from being given to a test object.

### Application Example 2

In the biological information measurement device according to the application example described above, it is preferable that detection of the biological information by the sensor section is intermittently performed.

According to such a biological information measurement device, the detection of biological information by the sensor section is intermittently performed at a necessary rate according to the situation of a test object by being controlled in the processing section. Therefore, the power supply which is supplied to the sensor section or the processing section is intermittently consumed. Therefore, it is possible to realize a biological information measurement device in which power consumption is suppressed, whereby it is possible to increase the time in which it is possible to continuously measure biological information.

### Application Example 3

In the biological information measurement device according to the application example described above, it is preferable that a time interval of performing detection is changed based on the detected biological information.

In such a biological information measurement device, a time interval of performing the detection of biological information by the sensor section can be changed. Therefore, it is possible to realize a biological information measurement device in which by extending the time interval of performing the detection based on the detected biological information, it is possible to increase measurable time while further suppressing power consumption.

### Application Example 4

In the biological information measurement device according to the application example described above, it is preferable that the biological information measurement device stores biological information of the test object at rest and when a change in the detected biological information is small compared to the biological information at rest, the time interval is extended, and when a change in the detected biological information is large, the time interval is shortened.

According to such a biological information measurement device, when a change in the detected biological information is small compared to the biological information at rest, it is possible to extend a time interval of detecting biological information. By extending the time interval, it is possible to suppress power consumption in the biological information measurement device. Further, when a change in the detected biological information is large compared to the biological information at rest, it is possible to shorten a time interval of detecting biological information. By shortening the time interval, it is possible to obtain a finer change in biological information. Therefore, it is possible to realize a biological information measurement device which is accurate and suppresses power consumption, thereby enabling measurement for a long period of time.

### Application Example 5

In the biological information measurement device according to the application example described above, it is preferable that the power supply section is provided with a rechargeable battery and the amount of charge of the battery is determined according to the number of times of detection or measurement time of the biological information performed after previous charging.

According to such a biological information measurement device, a rechargeable battery is used as the power supply section, and therefore, the mounting of the device main body on a test object is easy. Further, the amount of charge of the battery is determined according to the number of times of detection or measurement time of biological information performed after the previous charging, and therefore, it is possible to shorten charging time and to suppress deterioration of the battery. Therefore, it is possible to realize a biological information measurement device suitable for mounting on a test object.

### Application Example 6

In the biological information measurement device according to the application example described above, it is preferable that the amount of charge of the battery is set in advance according to a desired number of times of detection or measurement time and the charging of the battery is performed.

In such a biological information measurement device, by setting the amount of charge in advance according to the desired number of times of detection or measurement time and then performing the charging of the battery, it is possible to perform proper charging and perform the measurement of biological information even in a case where biological information is not obtained in advance or a case where a test object is different.

### Application Example 7

In the biological information measurement device according to the application example described above, it is preferable that the capacity of the battery to be installed is selected according to the number of times of the detection or the measurement time which is performed after the charging.

In such a biological information measurement device, the capacity of the battery to be installed is selected according to the number of times of the detection or the measurement time of biological information which is performed after the charging. That is, the capacity of the battery is selected according to the number of times of the detection of biological information which is desired on a single charge. Therefore, in a case where a measurement period is a short period of time and the number of times of detection is small, a battery having small capacity is selected, whereby it is possible to attain a reduction in the weight of the device main body which is mounted on a test obj ect and to alleviate a feeling of pressure to a test object. Further, in a case where a measurement period is a long period of time and the number of times of detection is large, a battery having large capacity is selected, whereby it is possible to continuously measure biological information of a test object, thereby obtaining finer biological information.

### Application Example 8

In the biological information measurement device according to the application example described above, it is preferable that the time interval of performing the detection is changed according to the amount of charge of the battery.

In such a biological information measurement device, a time interval of detecting biological information can be changed according to the amount of charge of the battery. Therefore, when there is a margin in the amount of charge of the battery, a time interval of performing detection is shortened, whereby it is possible to finely perform the detection of biological information when a change in biological information is large. Further, when the amount of charge of the battery is small, a time interval of performing detection is extended, whereby it is possible to measure biological information over a long period of time. Therefore, it is possible to detect biological information at the optimum time interval according to the amount of charge of the battery.

### Application Example 9

This application example of the invention is directed to a biological information measurement device including: a sensor section which detects biological information of a test object; a processing section which performs processing of the biological information; a power supply section which supplies electric power to the sensor section and the processing section; a device main body having a case section in which the sensor section, the processing section, and the power supply section are accommodated; and a band for mounting the device main body on the test object, wherein volume of the device main body of the biological information measurement device is less than or equal to 50 cc, and it is possible to measure biological information continuously for 10 hours or more.

According to such a biological information measurement device, it is possible to measure the biological information of a test object by the sensor section provided in the device main body mounted on the test object by the band. Further, the biological information measurement device is provided with the power supply section in which it is possible to supply a power supply capable of measuring biological information continuously for 10 hours or more, and the volume of the device main body is less than or equal to 50 cc. Therefore, when a test object moves, the device main body is suppressed from being caught in clothes, and thus it is possible to measure biological information with high precision continuously for 10 hours or more. Further, it is possible to realize a biological information measurement device in which, even if the biological information measurement device is mounted for a long period of time, a feeling of pressure or a feeling of fatigue is suppressed from being given to a test object.

### Application Example 10

In the biological information measurement device according to the application example described above, it is preferable that detection of the biological information by the sensor section is intermittently performed.

According to such a biological information measurement device, the detection of biological information by the sensor section is intermittently performed by being controlled in the processing section. Therefore, a power supply which is supplied to the sensor section or the processing section is intermittently consumed. Therefore, it is possible to realize a biological information measurement device in which power consumption is suppressed, whereby it is possible to increase the time in which it is possible to continuously measure biological information.

### Application Example 11

In the biological information measurement device according to the application example described above, it is preferable that a time interval of performing detection is changed based on the detected biological information.

In such a biological information measurement device, a time interval of performing the detection of biological information by the sensor section can be changed. Therefore, it is possible to realize a biological information measurement device in which by extending the time interval of performing the detection based on the detected biological information, it is possible to increase measurable time while further suppressing power consumption.

### Application Example 12

In the biological information measurement device according to the application example described above, it is preferable that the biological information measurement device stores biological information of the test object at rest and when a change in the detected biological information is small compared to the biological information at rest, the time interval is extended, and when a change in the detected biological information is large, the time interval is shortened.

According to such a biological information measurement device, when a change in the detected biological information is small compared to biological information at rest, it is possible to extend a time interval of detecting biological information. By extending the time interval, it is possible to suppress power consumption in the biological information measurement device. Further, when a change in the detected biological information is large compared to biological information at rest, it is possible to shorten a time interval of detecting biological information. By shortening the time interval, it is possible to obtain a finer change in biological information. Therefore, it is possible to realize a biological information measurement device which is accurate and suppresses power consumption, thereby enabling measurement for a long period of time.

### Application Example 13

This application example of the invention is directed to a biological information measurement device including: a sensor section which detects biological information of a test object; a processing section which performs processing of the biological information; a power supply section which supplies electric power to the sensor section and the processing section; a device main body having a case section in which the sensor section, the processing section, and the power supply section are accommodated; and a band for mounting the device main body on the test object, wherein the maximum thickness of the device main body of the biological information measurement device is less than or equal to 16 mm, and it is possible to measure biological information continuously for 10 hours or more.

According to such a biological information measurement device, it is possible to measure the biological information of a test object by the sensor section provided in the device main body mounted on the test object by the band. Further, the biological information measurement device is provided with the power supply section in which it is possible to supply a power supply capable of measuring biological information continuously for 10 hours or more, and the thickness of the device main body is less than or equal to 16 mm. Therefore, when a test object moves, the device main body is suppressed from being caught in clothes, and thus it is possible to measure biological information with high precision continuously for 10 hours or more. Further, it is possible to realize a biological information measurement device in which, even if the biological information measurement device is mounted for a long period of time, a feeling of pressure or a feeling of fatigue is suppressed from being given to a test object.

### Application Example 14

In the biological information measurement device according to the application example described above, it is preferable that detection of the biological information by the sensor section is intermittently performed.

According to such a biological information measurement device, the detection of biological information by the sensor section is intermittently performed by being controlled in the processing section. Therefore, a power supply which is supplied to the sensor section or the processing section is intermittently consumed. Therefore, it is possible to realize a biological information measurement device in which power consumption is suppressed, whereby it is possible to increase the time in which it is possible to continuously measure biological information.

### Application Example 15

In the biological information measurement device according to the application example described above, it is preferable that a time interval of performing detection is changed based on the detected biological information.

In such a biological information measurement device, a time interval of performing the detection of biological information by the sensor section can be changed. Therefore, it is possible to realize a biological information measurement device in which by extending the time interval of performing the detection based on the detected biological information, it is possible to increase measurable time while further suppressing power consumption.

### Application Example 16

In the biological information measurement device according to the application example described above, it is preferable that the biological information measurement device stores biological information of the test object at rest and when a change in the detected biological information is small compared to the biological information at rest, the time interval is extended, and when a change in the detected biological information is large, the time interval is shortened.

According to such a biological information measurement device, when a change in the detected biological information is small compared to biological information at rest, it is possible to extend a time interval of detecting biological information. By extending the time interval, it is possible to suppress power consumption in the biological information measurement device. Further, when a change in the detected biological information is large compared to biological information at rest, it is possible to shorten a time interval of detecting biological information. By shortening the time interval, it is possible to obtain a finer change in biological information. Therefore, it is possible to realize a biological information measurement device which is accurate and suppresses power consumption, thereby enabling measurement for a long period of time.

### BRIEF DESCRIPTION OF THE DRAWINGS

The invention will be described with reference to the accompanying drawings, wherein like numbers reference like elements.
Fig. 1 is a top view schematically showing the external appearance of a biological information measurement device according to an embodiment.
Figs. 2A and 2B are perspective views schematically showing the external appearance of the biological information measurement device according to the embodiment.
Fig. 3 is a development view schematically showing the structure of the biological information measurement device according to the embodiment.
Fig. 4 is an enlarged view schematically showing the structure of a main body section of the biological information measurement device.
Figs. 5A and 5B are side views schematically showing the nine o'clock-side side surface of the main body section of the biological information measurement device.
Figs. 6A and 6B are a plan view and a cross-sectional view schematically showing a first band portion.
Figs. 7A to 7C are a plan view and a cross-sectional view schematically showing a second band portion.

### DESCRIPTION OF EXEMPLARY EMBODIMENTS

Hereinafter, an embodiment of the invention will be described using the drawings. In addition, in each drawing shown below, in order to show each constituent element in size to the extent that can be recognized on the drawing, there is a case where the dimension or the ratio of each constituent element is described differently from that of the actual constituent element.

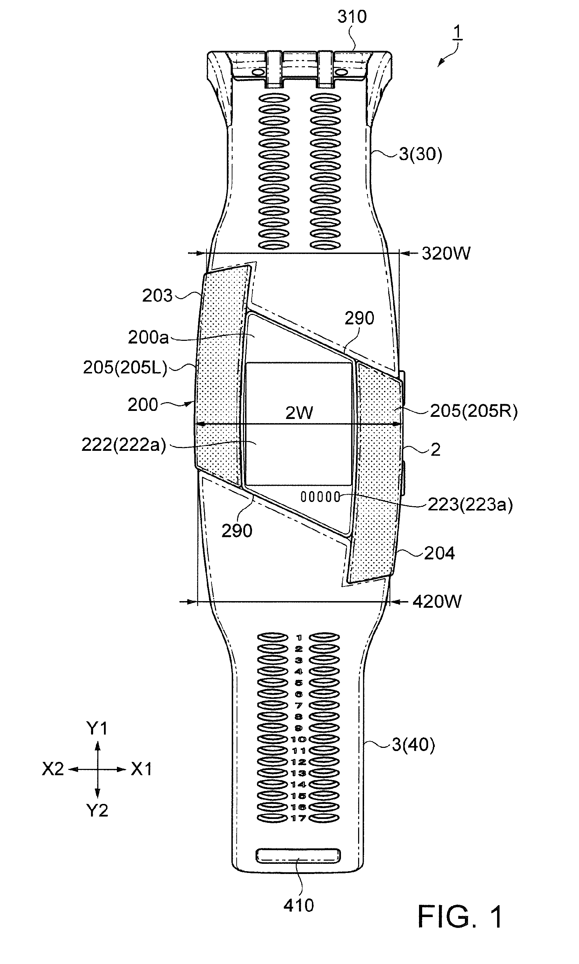

A biological information measurement device according to this embodiment will be described using Figs. 1 to 7C.

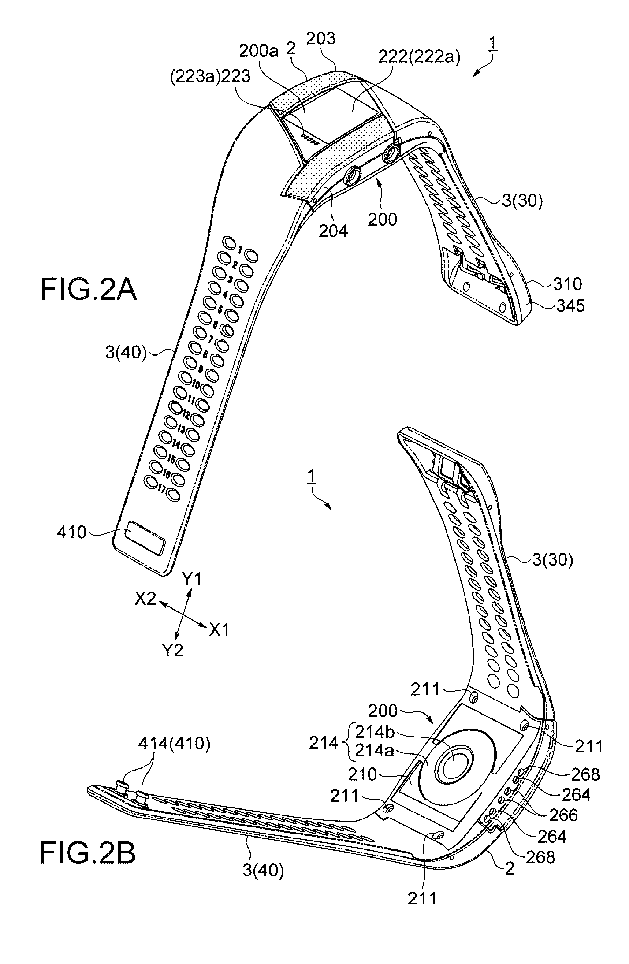

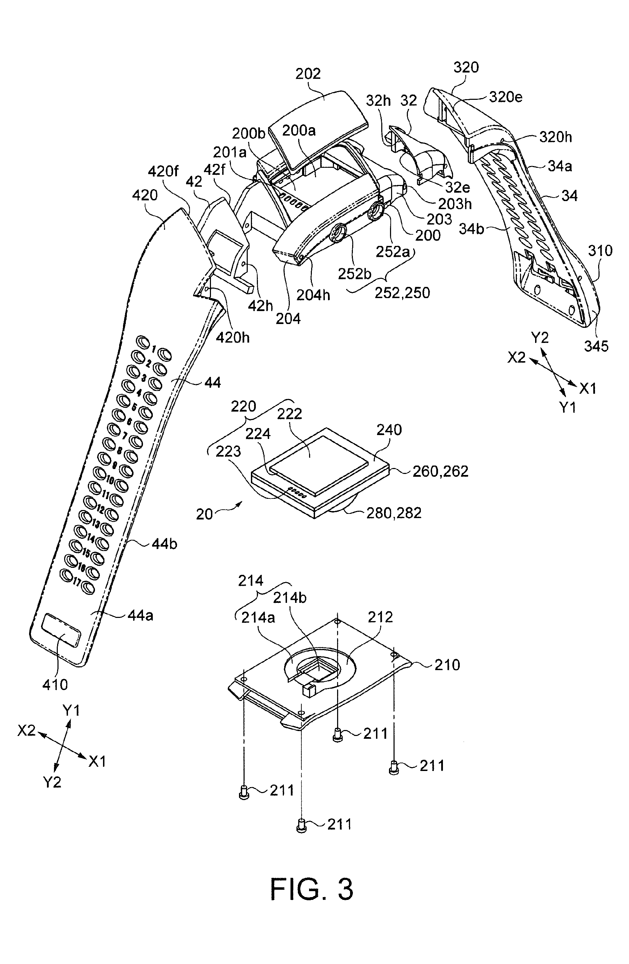

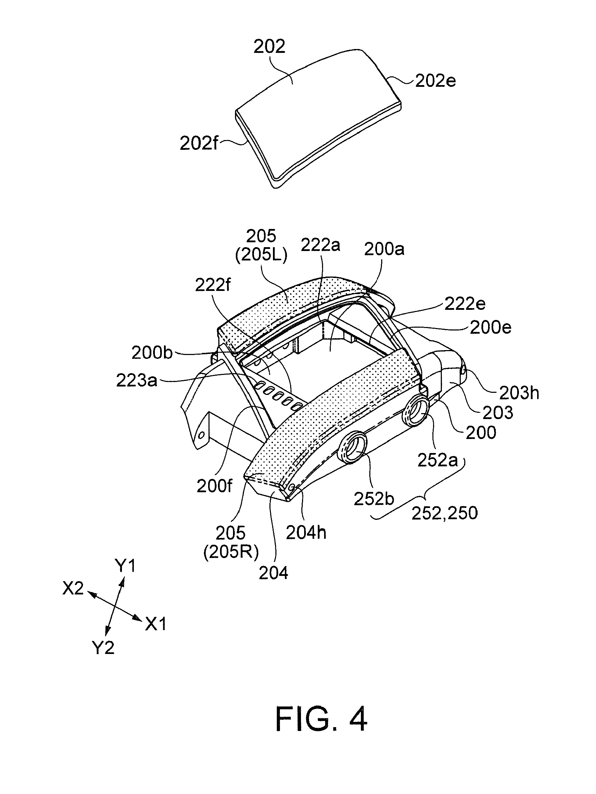

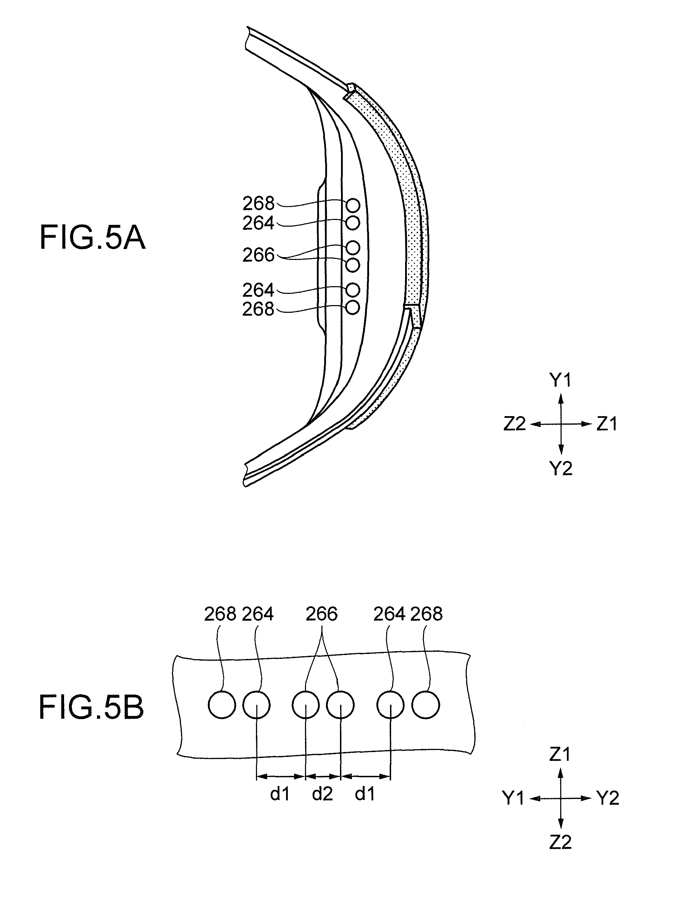

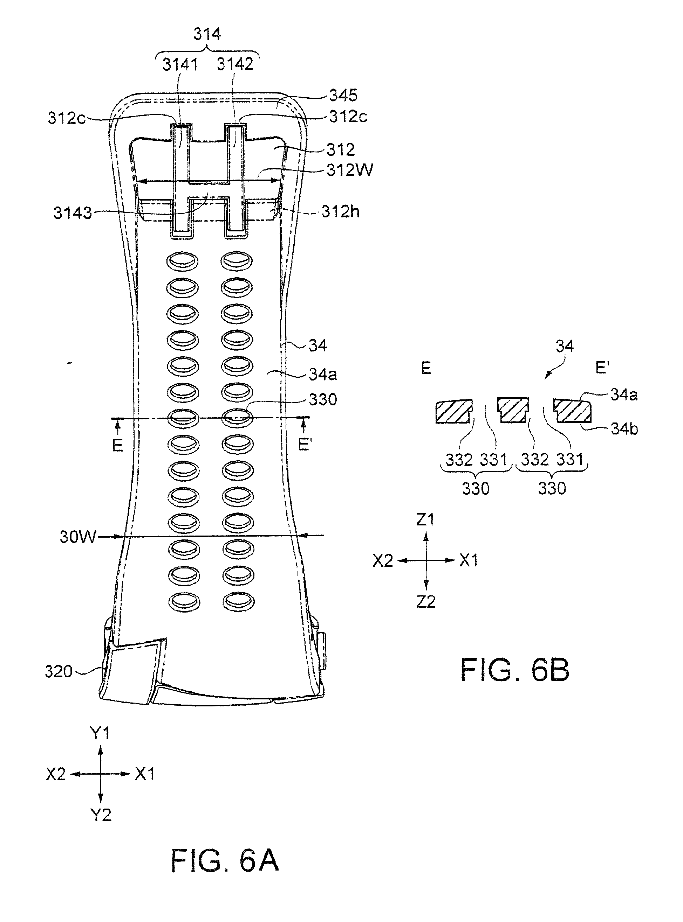

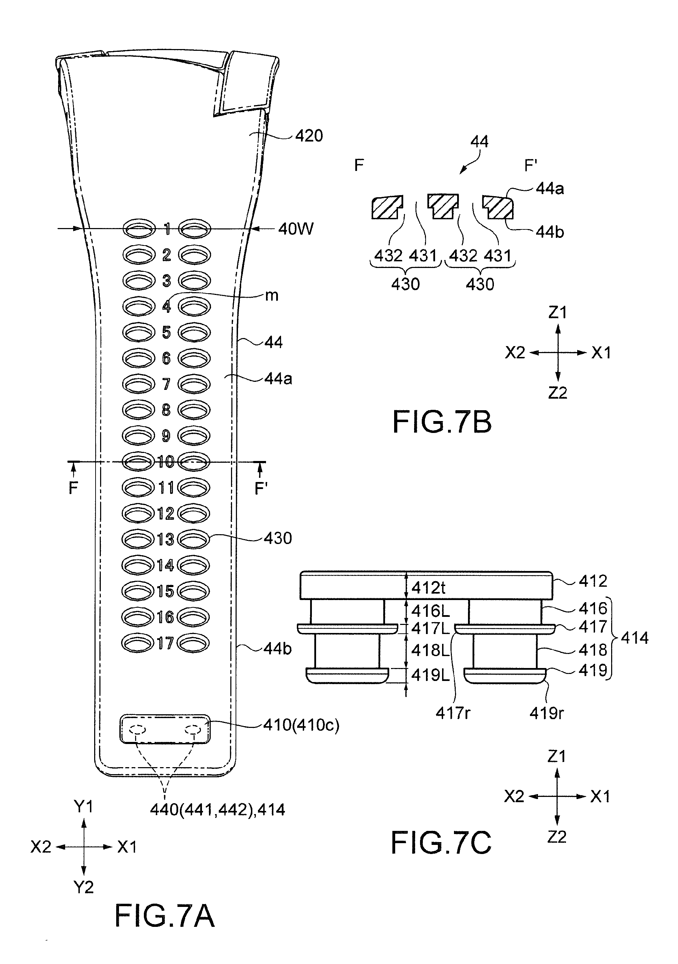

Fig. 1 is a top view schematically showing the external appearance of the biological information measurement device according to this embodiment. Figs. 2A and 2B are perspective views schematically showing the external appearance of the biological information measurement device. Fig. 3 is a development view schematically showing the structure of the biological information measurement device. Fig. 4 is an enlarged view schematically showing the structure of a main body section of the biological information measurement device. Figs. 5A and 5B are a side view schematically showing the nine o'clock-side side surface of the main body section of the biological information measurement device, and an enlarged view of a terminal section. Figs. 6A and 6B are a plan view and a cross-sectional view schematically showing a first band portion which is connected to a device main body. Figs. 7A to 7C are a plan view and a cross-sectional view schematically showing a second band portion which is connected to the device main body.

### Schematic Configuration of Biological Information Measurement Device 1

A biological information measurement device 1 (hereinafter referred to simply as a "measurement device 1") according to this embodiment is an electronic device which is mounted on the wrist or the like of a test object (for example, a human body), biological information of which is measured, and measures biological information such as the pulse. The measurement device 1 is formed to have an external appearance similar to a wristwatch, as shown in Figs. 1, 2A, and 2B. The measurement device 1 is provided with a device main body 2 which comes into close contact with a test object, thereby measuring biological information, and a band 3 which is mounted on the device main body 2.

### Configuration of Device Main Body 2

As shown in Figs. 2A, 2B, and 3, the device main body 2 of the measurement device 1 is provided with a module 20, and a case section 200 in which the module 20 is accommodated. The module 20 is provided with a display section 220, a processing section 240, a power supply section 260, and a sensor section 280.

Further, the device main body 2 is provided with an operating section 250 connected to the processing section 240, and a communication terminal 266. In addition, the device main body 2 is provided with a battery 262 connected to the power supply section 260, and a charging terminal 264 which is used in the charging of the battery 262.

In the case section 200, a concave portion 200b in which the module 20 is accommodated is formed on the lid section 210 side thereof. The module 20 is accommodated in the concave portion 200b and the lid section 210 is disposed so as to cover the concave portion 200b and is fixed by setscrews 211.

The lid section 210 can be made to have a concave shape in a shape facing the concave portion 200b. The lid section 210 is made to have a concave shape, whereby it is possible to vary the depth (the volume) of the concave portion 200b according to the thickness of the battery 262 (described later).

In this embodiment, the thickness of the device main body 2 is formed to be approximately 14 [mm]. When the thickness of the device main body 2 is thin, it is possible to alleviate a feeling of pressure which is given to a test object. Further, it is possible to suppress a measurement obstacle due to being caught in the clothes of a test object. It is preferable that the thickness of the device main body 2 is set to be greater than or equal to approximately 8 [mm] from the viewpoint of maintaining the strength thereof and to be less than or equal to approximately 16 [mm] from the viewpoint of being caught in the clothes of a test object or a feeling of pressure. The lid section 210 is made to have a concave shape in a range of the thickness, whereby it is possible to vary the thickness of the battery 262.

The volume of the device main body 2 is also varied by making the lid section 210 have a concave shape, as described above. In this embodiment, the volume of the device main body 2 is 21 [cc] in a case where the thickness of the device main body 2 is set to be 14 [mm]. In addition, it is preferable that the volume of the device main body 2 is set to be greater than or equal to approximately 15 [cc] from the viewpoint of maintaining the strength thereof and to be less than or equal to approximately 50 [cc] from the viewpoint of being caught in the clothes of a test object or a feeling of pressure.

Materials of the case section 200 and the lid section 210 are not particularly limited. In this embodiment, as an example thereof, nylon-based synthetic resin (plastic resin) in which the occurrence of cracks is hard is used.

As shown in Figs. 2A, 2B, and 3, in the case section 200, a display window 200a is provided on the opposite side to the side on which the lid section 210 is provided. The display window 200a is configured such that biological information or the like which is displayed on the display section 220 is visible.

The display window 200a is provided with a first display window 222a corresponding to a first display portion 222, and a second display window 223a corresponding to a second display portion 223. The first display portion 222 is fitted into the first display window 222a (refer to Figs. 1, 2A, and 2B). Further, the second display portion 223 is fitted into the second display window 223a (refer to Figs. 1, 2A, and 2B) .

As shown in Fig. 4, the display window 200a has a parallelogram shape in which an upper base (an upper side) 200e and a lower base (a lower side) 200f which are oblique sides extending in a direction (a second direction, an X direction shown in Figs. 1 to 3) intersecting a direction (a first direction, a Y direction shown in Figs. 1 to 3) in which the band 3 extends from the device main body 2 form a set of opposite sides.

The first display window 222a in which the first display portion 222 is fitted has a quadrangular shape having an upper base (an upper side) 222e and a lower base (a lower side) 222f which extend in the second direction intersecting the first direction in which the band 3 extends from the device main body 2.

The second display window 223a in which the second display portion 223 is fitted has a corner-rounded rectangular shape having parallel lines extending in the first direction in which the band 3 extends from the device main body 2.

Here, the display window 200a is disposed such that the upper base 200e of the display window 200a and the upper base 222e of the first display window 222a come into contact with each other at an acute angle. Further, the display window 200a is disposed such that the lower base 200f of the display window 200a and the lower base 222f of the first display window 222a come into contact with each other at an acute angle.

The second display window 223a is disposed at an area having an internal angle at which the lower base 200f of the display window 200a and the lower base 222f of the first display window 222a come into contact with each other at an acute angle.

The second display portions 223 and the second display windows 223a are provided at the area having the internal angle, whereby it is possible to display an operating state or the like of the measurement device 1 in a plurality of ways. Further, it is possible to make the first display portion 222 (the first display window 222a) be viewed as large when viewing the device main body 2, while suppressing the surface area of the device main body 2. Therefore, it is possible to suppress a feeling of pressure which is given to a person with the measurement device 1 mounted thereon.

Further, a display section cover body 202 formed of transparent resin, transparent glass, or the like is fitted into the display window 200a. The display section 220 is protected by the display section cover body 202. The display section cover body 202 is formed into a parallelogram shape based on the display window 200a. The display section cover body 202 has a parallelogram shape in which an upper base 202e and a lower base 202f which are oblique sides disposed in the first direction form a set of opposite sides.

As shown in Figs. 1 to 4, at the case section 200, a frame section 205 is provided along the display window 200a.

In the frame section 205, a frame portion 205R is provided in an X1 direction (the three o'clock-side direction when a direction in which a first band portion 30 extends is set to be the twelve o'clock direction in a timepiece) shown in Figs. 1 to 4 in the second direction intersecting the first direction in which the band 3 extends from the device main body 2, and a frame portion 205L is provided in an X2 direction (the nine o' clock-side direction when a direction in which a second band portion 40 extends is set to be the six o'clock direction in a timepiece).

The frame section 205 is disposed with the lower base thereof being in contact with the display window 200a (the display section cover body 202) and has an isosceles trapezoid shape in which a side becoming a leg is provided to extend in the second direction. Further, a side becoming the lower base of the frame section 205 is provided to be longer than a set of opposite sides extending in the first direction, which is different from a set of opposite sides in which the upper base 200e and the lower base 200f of the display window 200a make a pair.

In the frame portion 205L, a side becoming a leg disposed in an Y2 direction shown in Figs. 1 to 4 is provided on an extension line of the lower base 200f of the display window 200a. Therefore, in the frame portion 205L, a side becoming a leg provided in an Y1 direction shown in Figs. 1 to 4 is provided further to the Y1 side than an extension line of the upper base 200e of the display window 200a.

In the frame portion 205R, a side becoming a leg provided in the Y1 direction shown in Figs. 1 to 4 is provided on an extension line of the upper base 200e of the display window 200a. Therefore, in the frame portion 205R, a side becoming a leg provided in the Y2 direction is provided further to the Y2 side than an extension line of the lower base 200f of the display window 200a.

At the frame section 205, a film (not shown) which includes metal is provided on the front side of the case section 200 in which the display window 200a is provided. The film is electrically connected to the processing section 240 (described later). The film can be used as an antenna when performing wireless communication with an information processing device (not shown) provided outside the measurement device 1. Further, the film can be used as a so-called capacitance type touch sensor as the operating section 250 (described later). In addition, the film is provided, whereby it is possible to increase the strength of the case section 200 and thin the wall thickness of the case section 200. Therefore, it is possible to attain a reduction in the weight of the case section 200.

A material configuring the film is not particularly limited as long as it is a material capable of exhibiting performance as an antenna or a material capable of detecting a change in capacitance. In the measurement device 1 according to this embodiment, a material which includes nickel (Ni) is used. Nickel is used in the film, whereby it is possible to secure performance as an antenna, the strength of the case section 200, and performance as a touch switch and increase the design properties of the measurement device 1 by a glossy color which nickel has. In addition, in the measurement device 1 according to this embodiment, in order to increase performance as an antenna, the strength of the case section 200, and the design properties, the film is also provided on the side surface of the frame section 205.

As shown in Fig. 3, the display section 220 is provided in the module 20 accommodated in the case section 200. The first display portion 222 and the second display portion 223 are provided in the display section 220.

In the first display portion 222, biological information such as the number of pulses, time information such as the current time, or the like is displayed according to each display mode. Further, at the first display portion 222, a backlight 224 is provided and can illuminate the first display portion 222.

In the first display portion 222, as long as it is possible to display biological information (mainly, a numeral or a graph configured by a dot matrix) such as the pulse, a display method is not limited. In the measurement device 1, as an example thereof, a liquid crystal display device is used. Further, as for the backlight 224, as long as it is possible to perform illumination to the extent that biological information which is displayed on the first display portion 222 is visible, a luminescence method or a luminescent color is not limited. In the measurement device 1, the illumination of the first display portion 222 is performed by using an electro-luminescence (EL) panel which emits green light.

In the second display portion 223, each operation mode or the like of the measurement device 1 is displayed by the luminescent color or the blinking of the second display portion 223. In the second display portion 223, as long as it is possible to display each operation mode by the blinking or the luminescent color thereof, a display method is not limited. In the measurement device 1, as an example thereof, a light-emitting diode is used.

In addition, as the display section 220, a device (a liquid crystal panel or a light-emitting diode) which is used in the display of biological information or the like according to information to be transmitted is selected. In this way, it is possible to suppress the consumption of the battery 262 (described later) (power charged in the battery 262).

As shown in Fig. 3, the processing section 240 is provided in the module 20 accommodated in the case section 200.

The processing section 240 is a substrate configured by a semiconductor device such as a microcomputer, or a storage device, and an electronic circuit or the like which realizes a communication function and the charging control of the battery 262 connected to the power supply section 260. The display section 220, the operating section 250, the power supply section 260, the sensor section 280, and the film provided at the frame section 205 are connected to the processing section 240. The processing section 240 processes the driving of the sensor section 280 or a signal based on the pulse detected in the sensor section 280, thereby being able to perform the display processing of biological information or the like in the display section 220. Further, it is possible to analyze the signal based on the pulse detected in the sensor section 280, thereby determining an interval (a time interval) of detecting biological information. In addition, the processing section 240 performs the storing of the detected biological information and performs communication with the information processing device provided outside the device main body 2, thereby being able to output the stored data.

As shown in Fig. 3, the operating section 250 providing a command to the processing section 240 is provided in the module 20 accommodated in the case section 200.

In the operating section 250, an operation button 252 capable of being pressed is provided. By pressing the operation button 252, it is possible to perform the switching of a pulse measurement mode of displaying, for example, pulse measurement data, a clock mode of displaying the current time or the like, a remaining battery level display mode, a setting mode of setting an interval of detecting the pulse or the charging capacity of the battery 262, a lighting mode of the backlight 224 of the display section 220, or the like.

The operating section 250 is provided in the side surface of the case section 200 in the X1 direction (the three o'clock-side direction) shown in Figs. 1 to 4 in the second direction intersecting the first direction in which the band 3 extends from the device main body 2. The operating section 250 can be provided with a plurality of operation buttons 252. In the operating section 250, it is preferable that at least one operation button 252 is provided on the extension line of the upper base 200e of the display window 200a. In this way, a test object visually recognizes the upper base 200e of the display window 200a, whereby it is possible to easily grasp the position where the operation button 252 is provided, and thus it is possible to prevent an erroneous operation.

Further, by providing the plurality of operation buttons 252 and pressing the operation buttons 252 at substantially the same time, it is possible to perform switching to a setting mode of performing the setting of the measurement device 1. In the measurement device 1 according to this embodiment, as an example thereof, two operation buttons 252a and 252b are provided in the measurement device 1. An operation of the operating section 250 by a test object can be performed by pressing the operation button 252a by an index finger and adding a force associated with the pressing to the case section 200 through a thumb.

Further, an operation can be performed by pressing the operation button 252b by a ring finger and adding a force associated with the pressing to the case section 200 through a thumb. Therefore, it is preferable that an interval at which the operation buttons 252a and 252b are provided is an interval in which fingers (for example, a thumb and an index finger, or a thumb and a ring finger) performing the operation do not overlap, in a range that the fingers performing the operation reach.

As shown in Fig. 3, the power supply section 260 configuring the module 20 and serving as a power supply of the device main body 2 is provided in the case section 200. In addition, the battery 262 is provided in the power supply section 260. The battery 262 is provided as a rechargeable secondary battery. As the battery 262, a battery being small and lightweight and having high storage density is preferable. In addition, as long as it is a rechargeable secondary battery, there is no particular limitation, and a lithium-ion polymer battery, a lithium-ion battery, or the like can be used. In the measurement device 1 according to this embodiment, a lithium-ion battery (a coin type lithium-ion battery) being small and lightweight and having high energy density is used.

The charging terminal 264 which is used in the charging of the battery 262 is provided in the case section 200. In addition, the communication terminal 266 which is used in data communication of biological information (data) measured and stored in the measurement device 1, measurement setting data, and the like is provided in the case section 200.

As shown in Figs. 5A and 5B, the charging terminal 264 which is used in the charging of the battery 262 installed in the power supply section 260 which serves as the power supply of the measurement device 1 is provided in the case section 200. In addition, the communication terminal 266 which is used in data communication of biological information (data) measured and stored in the measurement device 1, the measurement setting data, or the like is provided in the case section 200.

The charging terminal 264 and the communication terminal 266 are provided in the side surface of the case section 200 in the X2 direction (the nine o'clock-side direction) shown in Figs. 1, 2A, and 2B in the second direction intersecting the first direction in which the band 3 extends from the device main body 2. Each of the charging terminal 264 and the communication terminal 266 is provided one or in a plurality in order to fulfill a function thereof. In the measurement device 1 according to this embodiment, as an example thereof, two charging terminals 264 and two communication terminals 266 are provided.

In the charging terminal 264 and the communication terminal 266, the communication terminals 266 are provided between the charging terminals 264. In other words, the charging terminal 264 is disposed outside the communication terminal 266 (in the Y direction shown in Fig. 5B). In addition, a positioning hole 268 for a connector (not shown) to be connected to the charging terminal 264 and the communication terminal 266 is provided outside each charging terminal 264 (in the Y direction which is the opposite direction to a direction in which the communication terminal 266 is provided) . In addition, each of the charging terminal 264 and the communication terminal 266 has a convex lens-shaped convex curved surface and is provided so as to protrude from the side surface of the case section 200. Further, the positioning hole 268 is provided as a bottomed hole in the side surface of the case section 200.

As shown in Fig. 5B, a distance d1 between the charging terminal 264 and the communication terminal 266 is wider than a distance d2 between the communication terminals 266. In this way, it is possible to suppress short circuits between the charging terminals 264 and between the charging terminal 264 and the communication terminal 266.

As for the charging terminal 264 and the communication terminal 266, it is possible to use a material having electric conductivity. In the measurement device 1 according to this embodiment, as an example thereof, stainless steel is used as materials of the charging terminal 264 and the communication terminal 266. In this way, the charging terminal 264 and the communication terminal 266 can have corrosion resistance. Further, the charging terminal 264 and the communication terminal 266 are provided to protrude from the case section 200 and each have a convex lens-shaped convex curved surface, and therefore, it is possible to easily perform cleaning, and thus it is possible to suppress corrosion of the charging terminal 264 and the communication terminal 266 and maintain electric conductivity. Further, the charging terminal 264 and the communication terminal 266 are provided in the side surface different from the side surface in which the operating section 250 is provided, and therefore, it is possible to suppress the fingers operating the operation buttons 252a and 252b from touching the terminals, thereby preventing contamination due to sebum or the like. In addition, in the charging terminal 264 and the communication terminal 266, by plating the surfaces with a conductive material having corrosion resistance, for example, gold or the like, it is possible to further improve the corrosion resistance.

Returning to Fig. 3, the sensor section 280 will be described. As shown in Fig. 3, the sensor section 280 provided with a sensor unit 282 is provided in the module 20 accommodated in the case section 200.

The measurement device 1 according to this embodiment can measure the pulse of a test object as one type of biological information. Therefore, the sensor unit 282 has a function to detect the pulse. Specifically, the sensor unit 282 is a light sensor and is provided with a sensor case, and a sensor substrate with a light emitting element and a light receiving element mounted thereon. The sensor unit 282 can measure the pulse that is the biological information of a test object by irradiating light toward the wrist of the test object from the light emitting element such as a light emitting diode (LED) and receiving the light reflected by a blood vessel of the wrist by the light receiving element such as a photodiode.

A sensor bank portion 212 in which the sensor section 280 is accommodated is provided at the lid section 210. The sensor section 280 is provided on the sensor bank portion 212 having a disk shape raised from the lid section 210 in the opposite direction to a display direction of the display section 220. The sensor bank portion 212 is provided with a sensor convex portion 214.

The sensor convex portion 214 is provided so as to be pressed against (be brought into contact with) the wrist or the like of a test object with the measurement device 1 mounted thereon. The sensor convex portion 214 is provided with a base portion 214a extending from the lid section 210, and a tip portion 214b which is pressed against a test object.

From this, it is preferable that the base portion 214a of the sensor convex portion 214 is configured with a material having a light shielding property. Further, it is preferable that the tip portion 214b is configured with a material capable of transmitting light which is emitted from the light emitting element. Further, it is preferable that the tip portion 214b has a shape which suppresses irregular reflection of the light and in which a test object does not feel pain at the time of mounting.

Therefore, the base portion 214a is provided by using the same synthetic resin as the lid section 210 and applying light-shielding coloring thereto. Further, the tip portion 214b is provided to have an arc shape by using transparent glass or transparent acrylic resin.

### Configuration of Band 3

The configuration of the band 3 will be described using Figs. 1 to 3, 6A, and 6B.

The band 3 is provided in order to mount the device main body 2 on a test object. The bands 3 are provided at both ends of the device main body 2, as shown in Figs. 1, 2A, and 2B. The first band portion 30 configuring the band 3 is mounted on a lug 203 (a lug on the twelve o'clock side in a timepiece as the first direction) of the device main body 2 by a mounting member 32, as shown in Fig. 3. Further, the second band portion 40 configuring the band 3 is mounted on a lug 204 (a lug on the six o'clock side in a timepiece as the first direction) of the device main body 2 by a mounting member 42.

A connection portion 310 which connects the first band portion 30 and the second band portion 40 is provided at the first band portion 30. The connection portion 310 is provided at an end portion of the first band portion 30 on the opposite side to the device main body 2 side. Further, a hook portion 410 which locks the second band portion 40 to the first band portion 30 is provided at the second band portion 40. The hook portion 410 is provided at an end portion of the second band portion 40 on the opposite side to the device main body 2 side.

In the following description, in the first band portion 30, the device main body 2 side is described as being referred to as "one end side" and the side on which the connection portion 310 is provided on the opposite side thereto is described as being referred to as the "other end side". Similarly, in the second band portion 40, the device main body 2 side is described as being referred to as "one end side" and the side on which the hook portion 410 is provided on the opposite side thereto is described as being referred to as the "other end side".

### Configuration of First Band Portion

The first band portion 30 shown in Figs. 3, 6A, and 6B has a belt portion 34, a cover portion 320 as a first connection portion (a connection portion) provided on one end side (the device main body 2 side, in the Y2 direction shown in Figs. 3 and 6A) of the belt portion 34, and the connection portion 310 on the other end side (the opposite side to the device main body 2, in the Y1 direction shown in Figs. 3 and 6A).

The belt portion 34 has front and back surfaces. In the following description, the surface coming into contact with a wrist when having been mounted on a test object is described as being referred to as a back surface 34b of the belt portion 34 , and the surface being the opposite surface thereto and being visible when having been mounted is described as being referred to as a front surface 34a of the belt portion 34.

### Mounting on Device Main Body 2

The first band portion 30 is mounted on the device main body 2 such that the cover portion 320 covers the lug 203, with the mounting member 32 sandwiched between the lug 203 and the cover portion 320.

The cover portion 320 and the lug 203 are pivotally supported by inserting a "spring rod" (not shown) into a hole 32h provided in the mounting member 32 and a "rod hole 203h" provided in the lug 203 and locking both ends of the spring rod to locking holes 320h provided in the cover portion 320.

Here, the connection between the device main body 2 and the first band portion 30 is performed with a miter joint (refer to Fig. 1) making the upper base 202e of the display section cover body 202 (the upper base 200e of the display window 200a) which is the oblique side of the device main body 2 and end portions 320e and 32e which are oblique sides of the cover portion 320 as the connection portion and the mounting member 32 face each other.

The mounting member 32 and the cover portion 320 are formed along the upper base 200e of the display window 200a provided in the case section 200. Further, in the measurement device 1, a bottomed groove 290 is provided between the first band portion 30 (the end portions 320e and 32e) and the device main body 2 (the upper base 200e) . The groove 290 is provided, whereby it is possible to suppress wear due to the contact of the case section 200 with the mounting member 32 and the cover portion 320 when the belt portion 34 (described later) expands or contracts.

### Belt Portion 34

The belt portion 34 shown in Figs. 6A and 6B has stretchability in order to mount the device main body 2 in close contact with the wrist or the like of a test object. The belt portion 34 is made using a material which includes polyurethane resin or silicone resin, thereby having stretchability and flexibility according to the characteristics of the material.

The belt portion 34 is provided with the thickness (in a Z direction shown in Fig. 6B) of a central portion in a direction of a width 30W increased in a cross-section (a cross-section in line E - E' shown in Fig. 6A) in a direction (the second direction, the X direction shown in Fig. 6A) intersecting a direction (the first direction, the Y1 direction shown in Fig. 6A) in which the first band portion 30 extends from the device main body 2. The belt portion 34 is provided with the thickness of the central portion in the direction of the width 30W increased, whereby strength at the time of expansion and contraction and strength at the time of flexion are secured.

A child hole 330 in which the hook portion 410 provided at the second band portion 40 is locked is provided in the belt portion 34. The child holes 330 are provided in parallel in the direction of the width 30W (the second direction, the X direction shown in Figs. 6A and 6B) and to be aligned to form rows in a direction (the first direction, the Y direction shown in Fig. 6A) in which the belt portion 34 extends.

In the child hole 330, as shown in Fig. 6B, a hole 331 provided on the front surface 34a side of the belt portion 34 and a hole 332 provided on the back surface 34b side of the belt portion 34 are coaxially provided.

The child hole 330 has an elliptical shape having a long axis (a major diameter) in the direction of the width 30W (the X direction) of the belt portion 34 and a short axis (a minor diameter) in an orthogonal direction (the Y direction) in which the first band portion 30 extends from the device main body 2. The long axis and the short axis orthogonal to the long axis of the child hole 330 are provided to be longer (larger) in the hole 332 than in the hole 331.

### Connection Portion 310

The connection portion 310 is provided on the other end side of the belt portion 34 (in the Y1 direction shown in Fig. 6A).

A thick portion 345 in which the thickness of the belt portion 34 is increased compared to a portion in which the child holes 330 are provided is provided on the other end side of the belt portion 34. The connection portion 310 is provided at the thick portion 345 in which the thickness of the belt portion 34 is increased. An insertion hole 312 into which the second band portion 40 is inserted and a projecting bar 314 which is inserted into a child hole 430 (refer to Figs. 7A to 7C) provided in the second band portion 40 are provided at the connection portion 310.

In the connection portion 310, the band insertion hole 312 is provided such that a width 312W in a direction (the X direction shown in Fig. 6A) intersecting a direction (the Y1 direction shown in Fig. 6A) in which the first band portion 30 extends from the device main body 2 is wider than a width 40W (refer to Fig. 7A) of the second band portion 40.

More specifically, a width on a side of one side (the Y1 side shown in Fig. 6A) on which a concave portion 312c (described later) is provided is provided to be wider than a width on the side of one side (the Y2 side shown in Fig. 6A) on which a locking hole 312h in which the projecting bar 314 (described later) is locked is provided.

The band insertion hole 312 is provided such that the width on the Y1 side is wider, whereby it is possible to easily insert the second band portion 40 therein. Further, it is possible to increase the close contact of the device main body 2 with a wrist by extending the second band portion 40 by pulling the second band portion 40 to the Y2 side with it being brought into contact with an edge of the band insertion hole 312 on the Y1 side on which the width is provided to be wide, and adjust tightening by the restoring force of the second band portion 40 (a belt portion 44).

The projecting bar 314 is pivotally supported on the belt portion 34 by a "spring rod" (not shown) which is inserted into the locking hole 312h provided at one side on the Y2 side at an inner edge of the band insertion hole 312, and a hole (not shown) provided in the projecting bar 314. A portion of the projecting bar 314 is fitted into the concave portion 312c provided at one side on the Y1 side at the inner edge of the band insertion hole 312, whereby the projecting bar 314 is locked to the belt portion 34.

In the projecting bar 314, two bar portions 3141 and 3142 are provided to extend in the Y1 direction from the side of one side on the Y2 side at the inner edge of the band insertion hole 312. Further, in the projecting bar 314, a connection bar 3143 is provided in the direction of the width 30W (the X direction shown in Fig. 6A) of the belt portion 34 intersecting the Y1 direction in which the bar portions 3141 and 3142 extend. The cross-sectional shape of each of the bar portions 3141 and 3142 configuring the projecting bar 314 has a flat shape. The projecting bar 314 has a flat shape, whereby it is possible to increase a contact area when having been inserted into the child hole 430, suppress the child hole 430 from being extended, and suppress a shift of the measurement device 1 mounted on a test object by the band 3 according to this embodiment.

A material configuring the projecting bar 314 is not particularly limited, and it is acceptable if it is a material capable of withstanding the restoring forces of the belt portions 34 and 44. In this embodiment, as for the projecting bar 314, as an example thereof, stainless steel is used. The projecting bar 314 can have toughness to the restoring forces of the belt portions 34 and 44 and corrosion resistance by using stainless steel. Further, the projecting bar 314 is subjected to hairline machining along a direction (the Y direction) in which the bar portions 3141 and 3142 extend. By performing the hairline machining, it is possible to increase the visibility of the projecting bar 314, thereby aiding insertion into the child hole 430.

In the band 3 according to this embodiment, the second band portion 40 is inserted into the band insertion hole 312 and the projecting bar 314 is inserted into the child hole 430 provided in the second band portion 40, whereby the first band portion 30 and the second band portion 40 are connected.

Here, it is required that the band 3 brings the device main body 2 into close contact with a test object in order to measure the biological information of the test object and the mounting position thereof is not shifted.

For this reason, the child holes 430 are provided in parallel in the second band portion 40, and the projecting bar 314 is inserted into the child hole 430, whereby a shift of a mounting position is suppressed (refer to Figs. 7A to 7C) . In the connection portion 310, the bar portions 3141 and 3142 configuring the projecting bar 314 are provided in parallel corresponding to the child holes 430 and an interval thereof is maintained by the connection bar 3143. In this way, the projecting bar 314 has an H-shape, and thus it is possible to easily insert the projecting bar 314 into the child holes 430 provided in parallel, while maintaining an interval between the bar portions 3141 and 3142 by the connection bar 3143.

### Cover Portion 320

The cover portion 320 as the first connection portion is provided on one end side (in the Y2 direction shown in Fig. 6A) of the belt portion 34. The locking hole 320h is provided in the cover portion 320.

The cover portion 320 is provided in order to connect the first band portion 30 to the device main body 2 through the mounting member 32. A width 320W of the cover portion 320 and a width 2W of the device main body 2 have approximately the same width (refer to Fig. 1).

More specifically, in a direction (the X direction shown in Figs. 1 to 3, or Figs. 6A and 6B) intersecting a direction (the Y1 direction shown in Figs. 1 to 6A) in which the first band portion 30 extends from the device main body 2, the width 320W of the cover portion 320 and the width 2W of the device main body 2 have approximately the same width. Further, the cover portion 320 is formed into a shape having the end portion 320e which becomes an oblique side along the upper base 200e of the display window 200a formed into a parallelogram shape (refer to Fig. 3). In this way, the device main body 2 and the first band portion 30 can be visible as being visually integrally formed, and thus it is possible to alleviate a feeling of pressure due to mounting. Further, since the device main body 2 does not protrude from the first band portion 30 in the width direction (the X direction), it is possible to suppress the clothes of a test object from being caught in the device main body 2 at the time of mounting.

### Configuration of Second Band Portion

The second band portion 40 shown in Figs. 3, and 7A to 7C has the belt portion 44, the cover portion 420 as a second connection portion (a connection portion) provided on one end side (the device main body 2 side, in the Y1 direction shown in Figs. 3 and 7A) of the belt portion 44, and the hook portion 410 on the other end side (the opposite side to the device main body 2, in the Y2 direction shown in Figs. 3 and 7A). In addition, in Fig. 7A, illustration of a concave portion 410c in which the hook portion 410 is provided and a child hole 440 is simplified.

The belt portion 44 has front and back surfaces, and in the following description, the surface coming into contact with a wrist when having been mounted on a test object is described as being referred to as a back surface 44b of the belt portion 44 and the surface being the opposite surface thereof and being visible when having been mounted is described as being referred to as a front surface 44a of the belt portion 44.

### Mounting on Device Main Body 2

The second band portion 40 is mounted on the device main body 2 such that the cover portion 420 covers the lug 204, with the mounting member 42 sandwiched between the lug 204 and the cover portion 420.

The cover portion 420 and the lug 204 are pivotally supported by inserting a "spring rod" (not shown) into a hole 42h provided in the mounting member 42 and a "rod hole 204h" provided in the lug 204 and locking both ends of the spring rod to locking holes 420h provided in the cover portion 420.

Here, the connection between the device main body 2 and the second band portion 40 is performed with a miter joint (refer to Fig. 1) making the lower base 202f of the display section cover body 202 (the lower base 200f of the display window 200a) which is the oblique side of the device main body 2 and end portions 420f and 42f which are oblique sides of the cover portion 420 as the connection portion and the mounting member 42 face each other.

The mounting member 42 and the cover portion 420 are formed along the lower base 200f of the display window 200a provided in the case section 200. Further, in the measurement device 1, the bottomed groove 290 is provided between the second band portion 40 (the end portions 420f and 42f) and the device main body 2 (the lower base 200f). The groove 290 is provided, whereby it is possible to suppress wear due to the contact of the case section 200 with the mounting member 42 and the cover portion 420 when the belt portion 44 (described later) expands or contracts.

### Belt Portion 44

The belt portion 44 shown in Figs. 7A and 7B has stretchability in order to mount the device main body 2 in close contact with the wrist or the like of a test object. The belt portion 44 is made using a material which includes polyurethane resin or silicone resin, thereby having stretchability and flexibility according to the characteristics of the material.

The belt portion 44 is provided with the thickness (in the Z direction shown in Fig. 7B) of a central portion in a direction of the width 40W increased in a cross-section (a cross-section in line F - F' shown in Fig. 7A) in a direction (the second direction, the X direction shown in Fig. 7A) intersecting a direction (the first direction, the Y2 direction shown in Fig. 7A) in which the second band portion 40 extends from the device main body 2. The belt portion 44 is provided with the thickness of the central portion in the direction of the width 40W increased, whereby strength at the time of expansion and contraction and strength at the time of flexion are secured.

The child hole 430 in which the projecting bar 314 provided at the first band portion 30 is inserted is provided in the belt portion 44. The child holes 430 are provided in parallel in the direction of the width 40W (the second direction, the X direction shown in Figs. 7A to 7C) and to be aligned to form rows in a direction (the first direction, the Y direction shown in Figs. 7A and 7B) in which the belt portion 44 extends.

In the child hole 430, as shown in Fig. 7B, a hole 431 provided on the front surface 44a side of the belt portion 44 and a hole 432 provided on the back surface 44b side of the belt portion 44 are coaxially provided.

The child hole 430 has an elliptical shape having a long axis (a major diameter) in the direction of the width 40W (the X direction) of the belt portion 44 and a short axis (a minor diameter) in an orthogonal direction (the Y direction) in which the second band portion 40 extends from the device main body 2, as shown in Fig. 7B. The long axis and the orthogonal short axis of the child hole 430 are provided to be longer (larger) in the hole 432 than in the hole 431.

In addition, the elliptical shape that the holes 431 and 432 have is a shape according to the cross-sectional shape of the projecting bar 314 provided at the connection portion 310 described above and may be appropriately changed in accordance with the cross-sectional shape of the projecting bar 314. The holes 431 and 432 have the elliptical shape, thereby being able to be easily deformed, and thus the insertion of the projecting bar 314 can be easily performed. In addition, the holes 431 and 432 are also excellent in a restoring force after the deformation.

In the belt portion 44, as shown in Fig. 7A, symbol m is denoted between the child holes 430 provided in parallel. The symbols m are denoted to form order toward the direction (the Y2 direction shown in Fig. 7A) in which the second band portion 40 extends from the device main body 2. The symbols m are denoted as "1, 2, 3, ... , and 17" in order from the device main body 2 side (the Y1 side shown in Fig. 7A) in a case of being, for example, a numeral. Further, in a case of being an alphabet letter, the symbols can be denoted as "a, b, c, ..." in order from the device main body 2 side. In a case of using an alphabet letter, compared to a case of using a numeral, it is possible to indicate a permutation of a lot of child holes 430 with the same letter size. That is, it is possible to indicate a permutation of the child holes 430 of the number equivalent to the twenty-five letters of the alphabet with one letter while maintaining the size of the letter. The font of the symbol m is not particularly limited as long as it is possible to indicate the order of the child holes 430.

The symbols m are denoted on the belt portion 44, whereby, when repeatedly mounting the measurement device 1 on a wrist or the like, it is possible to easily identify the optimum position for inserting the projecting bar 314 in order to connect the first band portion 30 and the second band portion 40, and thus it is possible to mount the measurement device 1 without seeking the position of the child hole 430 for inserting the projecting bar 314 which is optimum for each mounting. Further, the symbol m is denoted between the child holes 430 provided in parallel, and therefore, it is possible to denote the symbol to the second band portion 40 without newly providing a space (an area).

Further, as shown in Fig. 7A, a concave portion (not shown) in which a base portion 412 of the hook portion 410 (described later) is fitted and the child hole 440 in which a pin 414 extending from the base portion 412 is inserted are provided in the belt portion 44.

The concave portion has a concave shape conforming to an outer peripheral edge of the base portion 412 on the front surface 44a of the belt portion. Further, a depth of the concave portion is provided to be approximately the same dimension as a thickness 412t of the base portion 412.

The child holes 440 are provided in parallel in the direction of the width 40W (the X direction) and coaxially with the pins 414 extending from the base portion 412, as shown in Fig. 7A. In the child hole 440, a hole (not shown) provided in a bottom portion of the concave portion on the front surface 44a side of the belt portion and a hole (not shown) provided in the back surface 44b of the belt portion are coaxially provided.

### Hook Portion 410

As shown in Figs. 1 to 3 or Figs. 7A to 7C, the hook portion 410 is provided on the other end side (in the Y2 direction shown in Figs. 1 to 3 or Fig. 7A) of the belt portion 44.

As shown in Fig. 7C, the hook portion 410 is provided with the base portion 412 and the pins 414 extending from the base portion 412. The pin 414 is configured to include a first shaft portion 416 and a second shaft portion 418. The hook portion 410 is a member for locking the second band portion 40 inserted into the connection portion 310 to the first band portion 30 by inserting the pins 414 into the child holes 330 provided in the first band portion 30.

In the hook portion 410, the plurality of pins 414 are provided in accordance with an interval of the child holes 330 provided in parallel in the direction of the width 30W (the X direction shown in Figs. 1 to 3) of the belt portion 34.

Since the pin 414 is inserted into the child hole 440 provided in the second band portion 40 and the child hole 330 of an elliptical shape provided in the first band portion 30, the cross-sectional shape thereof is an elliptical shape. The pin 414 is formed into an elliptical shape having a long axis (a major diameter) in the direction of the width 30W of the belt portion 34 (the direction of the width 40W of the belt portion 44) and a short axis (a minor diameter) in an orthogonal direction (the Y direction) in which the second band portion 40 extends from the device main body 2.

In the hook portion 410, the first shaft portion 416 configuring the pin 414 is provided so as to protrude from the base portion 412. Further, the second shaft portion 418 is provided at one end (in a Z2 direction shown in Fig. 7C, one end on the opposite side to the base portion 412 from which the first shaft portion 416 extends) of the first shaft portion 416.

A first head portion 417 is provided at the first shaft portion 416. The first head portion 417 is provided on the opposite side (in the Z2 direction shown in Fig. 7C) to the base portion 412 from which the first shaft portion 416 extends. The first head portion 417 has a curved surface 417r at an outer peripheral edge on the side (in the Z2 direction shown in Fig. 7C) on which the second shaft portion 418 is provided.

The diameter of the first head portion 417 is provided to be larger than the diameter of the first shaft portion 416. Further, the diameter of the first head portion 417 is provided to be larger than the diameter of a hole 441 and smaller than the diameter of a hole 442.

Further, a second head portion 419 is provided at the second shaft portion 418. The second head portion 419 is provided on the opposite side (in the Z2 direction shown in Fig. 7C) to the first shaft portion 416 from which the second shaft portion 418 extends. The second head portion 419 has a curved surface 419r at an outer peripheral edge on the opposite side (in the Z2 direction shown in Fig. 7C) to the side on which the first shaft portion 416 is provided.

The diameter of the second head portion 419 is provided to be larger than the diameter of the second shaft portion 418.

Further, the diameter of the first head portion 417 is provided to be larger than the diameter of the hole 331 provided in the child hole 330 in which the second head portion 419 is inserted and smaller than the diameter of the hole 332.

Here, the thickness (in a direction in which the pin 414 extends, the thickness in the Z direction shown in Fig. 7C) of the base portion 412 and the length (in the direction in which the pin 414 extends, the length in the Z direction shown in Fig. 7C) of the pin 414 are determined depending on the depths of the child hole 440 and the hole 331 (the child hole 330).

The thickness 412t of the base portion 412 in this embodiment is approximately the same as the depth of the concave portion. A length 416L of a shaft portion of the first shaft portion 416 is approximately the same as the depth (the length) of the hole 441. A length (a thickness) 417L of the first head portion 417 of the first shaft portion 416 is approximately the same as the depth (the length) of the hole 442. A length 418L of a shaft portion of the second shaft portion 418 is approximately the same as the depth (the length) of the hole 331. A length (a thickness) 419L of the second head portion 419 of the second shaft portion 418 is approximately the same as the depth (the length) of the hole 332.

In the hook portion 410, the base portion 412 is fitted into the concave portion 410c provided in the front surface 44a of the belt portion 44. In addition, the pins 414 extending from the base portion 412 are inserted into the child holes 440 provided in parallel in the direction of the width 40W of the belt portion 44, whereby the hook portion 410 is provided at the belt portion 44. The hook portion 410 is provided such that the second shaft portions 418 protrude from the back surface 44b of the belt portion 44 when having been provided at the belt portion 44. The second shaft portions 418 protrude from the belt portion 44, thereby being able to be inserted into the child holes 330 provided in the first band portion 30 (the belt portion 34).

In this way, in the first shaft portion 416 inserted into the hole 441, the first head portion 417 is caught in the hole 441, and thus it is possible to suppress the hook portion 410 from being detached from the belt portion 44.

More specifically, due to the curved surface 417r provided at the first head portion 417, it is possible to reduce contact resistance occurring when inserting the first shaft portion 416 into the hole 441. In addition, the curved surface 417r is not provided at the first head portion 417 on the base portion 412 side, and therefore, when extracting the first shaft portion 416 from the hole 441, a larger force than the force at the time of insertion is required. Therefore, it is possible to suppress the hook portion 410 from being detached from the belt portion 44.

Further, in the second shaft portion 418 inserted into the child hole 330, the second head portion 419 is caught in the hole 331 provided in the first band portion 30, and thus it is possible to lock the second band portion 40 to the first band portion 30. Further, it is possible to suppress detachment of the second band portion 40 locked to the first band portion 30.

More specifically, due to the curved surface 419r provided at the second head portion 419, it is possible to reduce contact resistance occurring when inserting the second shaft portion 418 into the hole 331. In addition, the curved surface 419r is not provided at the second head portion 419 on the first shaft portion 416 side, and therefore, when extracting the second shaft portion 418 from the hole 331, a larger force than the force at the time of insertion is required. Therefore, it is possible to suppress detachment of the second band portion 40 locked to the first band portion 30.

### Cover Portion 420

The cover portion 420 as the second connection portion is provided on one end side (in the Y1 direction shown in Fig. 7A) of the belt portion 44. The locking hole 420h is provided in the cover portion 420.

The cover portion 420 is provided in order to connect the second band portion 40 to the device main body 2 through the mounting member 42. A width 420W of the cover portion 420 and the width 2W of the device main body 2 have approximately the same width (refer to Fig. 1).

More specifically, in a direction (the X direction shown in Figs. 1 to 3, or Figs. 7A to 7C) intersecting the direction (the Y1 direction shown in Figs. 1 to 3, or Fig. 7A) in which the second band portion 40 extends from the device main body 2, the width 420W of the cover portion 420 and the width 2W of the device main body 2 have approximately the same width. Further, the cover portion 420 is formed into a shape having the end portion 420f which becomes an oblique side along the lower base 200f of the display window 200a formed into a parallelogram shape (refer to Fig. 3). In this way, the device main body 2 and the second band portion 40 can be visible as being visually integrally formed, and thus it is possible to alleviate a feeling of pressure due to mounting. Further, since the device main body 2 does not protrude from the second band portion 40 in the width direction (the X direction), it is possible to suppress the clothes of a wearer from being caught in the device main body 2 at the time of mounting.

### Operation of Device Main Body 2

The device main body 2 can detect the pulse which is one type of biological information of a test object by the sensor section 280. The detection of the biological information (the pulse) can be performed by making the sensor unit 282 provided in the sensor section 280 function intermittently.

The biological information detected by the sensor unit 282 is transmitted to the processing section 240 and various information processing is performed thereon. The biological information subjected to the information processing is displayed on the display section 220 or recorded in a storage device provided in the processing section 240. The recorded biological information can be transmitted to the outside of the device main body 2 by wireless communication between the device main body 2 and a receiving device (not shown) configuring the measurement device 1 or wire communication through the communication terminal 266.

Here, in biological information such as the pulse, the value thereof and a change in value associated with the passage of time are significantly different from each other according to the activity of a test object. For example, in the pulse, when a test object is sleeping, a change in value associated with the passage of time is small. On the other hand, when a test object is in motion (walking, jogging, or the like), a change in value associated with the passage of time is large.

In a case of measuring the pulse as biological information, it is important to catch a change in pulse with time (the passage of time). Therefore, even if the pulse is detected continuously or in a short period when a change in pulse with time is small, utility value is low as biological information. Further, even if the pulse is detected intermittently when a change in pulse with time is large, it is not possible to capture a change in pulse, and thus useful biological information is not obtained.

If the measurement of biological information is continuously performed, these problems are solved. However, the battery 262 installed in the device main body 2 is quickly consumed, and thus a problem in that a period when it is possible to measure biological information is shortened occurs newly.

Therefore, in the measurement device 1 according to this embodiment, an interval of detecting the pulse in the device main body 2 can be changed based on the detected pulse. That is, in a case where a change in biological information is small, a detection interval is extended, and in a case where a change in biological information is large, a detection interval is shortened.

Specifically, the pulse detected in the sensor section 280 is analyzed in the processing section 240, and if the pulse of a test object is less than a predetermined value (for example, a pulse value at rest) and a variation of the pulse in a predetermined period of time is in a range of a predetermined change rate, a detection interval of the pulse by the sensor section 280 is increased. Further, the pulse detected in the sensor section 280 is analyzed in the processing section 240, and if the pulse exceeds a predetermined value and the pulse exceeds a range of a predetermined change rate in a predetermined period of time, a detection interval of the pulse by the sensor section 280 is shortened.

In addition, a detection interval in the measurement device 1 includes an aspect in which a mode of continuously measuring biological information, a mode of displaying time on the display section 220, a mode in which display in the display section 220 is not performed, and the like are combined, in addition to only an operation interval of the sensor section 280.

In the measurement device 1 according to this embodiment, by analyzing the biological information of a test object in the processing section 240, it is possible to obtain a detection interval (the number of times of detection according to time) of biological information needed for, for example, one day (24 hours). Further, it is possible to obtain a detection interval (the number of times of detection according to time) of biological information according to activity such as a daytime activity zone or a night-time sleeping zone, for example.

In addition, with respect to a predetermined value determining a pulse detection interval and a change of the detection interval, and a change rate, a proper value is different according to a test object on which the measurement device 1 is mounted. Therefore, it is possible to set the value at an arbitrary value according to a test object by using the operating section 250.

Therefore, consumption of the battery 262 is suppressed by halting the detection operation of the sensor section 280 when a change in pulse (biological information) is small, and thus it is possible to extend the measurement period of biological information by a single charge.

Power consumption (current consumption) in the measurement device 1 according to this embodiment will be described.

In a case of continuously measuring the pulse as biological information (at a sampling rate of 15 Hz, for example) in the measurement device 1 (hereinafter referred to as a "detection mode"), current consumption of approximately 1400 [µA·H] occurs. Further, in a case of halting the measurement of biological information and then displaying time or the like in the measurement device 1 (including a certain operation by the operating section 250, lighting of the backlight 224, and the like, hereinafter referred to as a "time display mode"), current consumption of approximately 4 60 [µA·H] occurs. Further, in a case of halting the measurement of biological information in the measurement device 1 and not being mounted on a test object (a case where there is no operation by the operating section 250, or the like, hereinafter referred to as a "standby mode"), current consumption of approximately 350 [µA·H] occurs. Further, in a case where display of time or the like in the display section 220 is not performed (hereinafter referred to as a "non-display mode"), current consumption of approximately 14 [µA·H] occurs.

Here, in a case where the capacity of the battery 262 (a coin type lithium-ion battery, model PD2032 is illustrated) which is installed is Typ45 [mA·H] (nominal 75 [mA·H]), in a case of continuing the detection mode, it is possible to continue it for approximately 32 hours. Further, in a case of continuing the time display mode, it is possible to continue it for approximately 98 hours. Further, in a case of continuing the standby mode, it is possible to continue it for approximately 129 hours. Further, in a case of continuing the non-display mode, it is possible to continue it for approximately 3214 hours.

In the measurement device 1 according to this embodiment, a detection interval (including the combination of the respective modes described above) of detecting the pulse can be changed based on the pulse that is biological information detected as described above.

For example, an operation of the measurement device 1 is performed at the detection mode in a case where a test object is in motion. Further, the measurement device 1 operates with the ratio between the detection mode and the time display mode being 5:5 in a case where a test object is performing light work or the like. Further, the measurement device 1 operates with the ratio between the detection mode and the standby mode being 2 : 8 when a test object is at a resting state such as sleeping. Here, it is assumed that time when a test object is in motion is 8 hours, time when a test object is performing light work is 8 hours, and time in a resting state is 8 hours.

Power (energy) needed in order to measure biological information associated with the activity of a test object described above is subjected to current consumption of 11600 [µA·H] for 8 hours when a test object is in motion, 9600 [µA·H] for 8 hours when light work is being performed, and 8484 [µA·H] for 8 hours in a resting state, a total of 29684 [µA·H]. Therefore, it is possible to perform the measurement of the biological information of a test object continuously for 24 hours or more on a single charge.

In addition, in the measurement device 1, if the measurement of biological information can be performed continuously for 24 hours or more, it is possible to obtain biological information in one-day activity (for example, one cycle at the time of daytime activity and the time of night-time sleep) of a test object. Further, if the measurement of biological information can be performed continuously for 36 hours or more, it is possible to obtain biological information at the time of two-day's activity (for example, the time of daytime activity, the time of night-time sleep, and the time of next day's activity) of a test object. Further, if the measurement of biological information can be performed continuously for 48 hours or more, it is possible to obtain biological information in the activity for two days of a test object.

An operation ratio of the measurement device 1 described above may be appropriately changed based on biological information obtained from a test object. By changing an operation ratio of the measurement device 1, it is possible to extend time when it is possible to perform the measurement of biological information continuously without changing the capacity of the battery 262 or the number of batteries 262.

In addition, it is possible to extend time when it is possible to perform the measurement of biological information continuously by changing the capacity of the battery 262 or the number of batteries 262 without changing an operation ratio of the measurement device 1. An increase in the capacity of the battery 262 can be performed in a range in which the thickness of the device main body 2 described above does not exceed 16 [mm].

Further, in the measurement device 1, it is possible to extend a detection interval of biological information (change an operation ratio) according to the remaining capacity of the battery 262.

When the processing section 240 determines that the measurement of biological information in a measurement period set in advance cannot be achieved due to the capacity of the battery 262, it is possible to extend an interval of detecting the pulse by using the sensor section 280 according to the capacity of the battery 262. In this way, even if the action of a test object is different from the content set in advance, it is possible to prevent missing of the measurement of biological information in a measurement period set in advance. Therefore, it is possible to objectively analyze the lifestyle of a test object by performing the measurement of biological information continuously over a long period of time.

### Charging of Device Main Body 2

In the measurement device 1 according to this embodiment, it is possible to measure biological information by mounting the device main body 2 on a test object by the band 3. For this reason, the device main body 2 is equipped with the rechargeable battery 262 (a secondary battery) as a drive source. The charging of the battery 262 is performed in a state where the device main body 2 is not mounted on a test object. Specifically, power for charging is supplied by connecting a connector (not shown) to the charging terminal 288 provided in the side surface of the case section 200. Further, the control of the charging of the battery 262 can be performed in a charging control circuit provided in the processing section 240.

Here, it is required that the amount of charge of the battery 262 and the capacity of the battery 262 are equal to the power (energy) amount according to the number of times of detection (a measurement period) of biological information.

In the charging of the battery 262, for example, in a case of performing the measurement of biological information for 24 hours (one day), the charging of power according to a detection interval (the number of times of detection) needed for 24 hours is performed. Current consumption in a case of performing the measurement of biological information continuously for 24 hours is approximately 33600 [µA·H] (approximately 1400 [µA·H] per hour). Therefore, the amount of charge of the battery 262 is set to be greater than or equal to 33600 [µA·H] at which it is possible to perform the measurement of biological information continuously for 24 hours. Further, for example, in a case of performing the measurement of biological information in only a daytime activity time zone, the charging of a power amount according to a detection interval (the number of times of detection) needed for a daytime activity time zone is performed. Current consumption in a case of performing the measurement of biological information continuously during daytime for 8 hours is approximately 11200 [µA·H] (approximately 1400 [µA·H] per hour). Therefore, the amount of charge of the battery 262 is set to be greater than or equal to 11200 [µA·H] at which it is possible to perform the measurement of biological information continuously for 8 hours. Further, the amount of charge of the battery 262 can be controlled based on biological information analyzed in the processing section 240. That is, the amount of charge of the battery 262 can be set to be the amount of charge in which it is possible to perform measurement at a detection interval according to the activity of a test object analyzed in the processing section 240.

In this way, the amount of charge can be set to be the amount of charge of the battery 262 needed in a period (time) of measuring biological information. Therefore, it is possible to shorten charging time and to suppress deterioration of the battery 262.

The capacity of the battery 262 is selected according to measurement time and a detection interval of biological information. For example, in a case of the aim being the measurement of biological information in an activity time zone, it is possible to select the battery 262 having a capacity according to a detection interval (the number of times of detection) needed in the activity time zone. For example, in a case of aiming at the measurement of biological information in a sleeping time zone, it is possible to select the battery 262 having a capacity according to a detection interval (the number of times of detection) needed in the sleeping time zone. That is, by varying the capacity of the battery 262 according to a test object state (a time zone) in which the measurement of biological information is desired, it is possible to attain a reduction in the weight of the device main body 2. In addition, by varying the size of the case section 200 of the device main body 2 according to the electrical capacity and the physical volume of the battery 262, it is possible to attain a reduction in the size of the device main body 2.

According to the embodiment described above, the following effects can be obtained.

According to the measurement device 1, it is possible to measure the biological information of a test object by the sensor section 280 provided in the device main body 2 mounted on the test object by the band 3. The detection of biological information by the sensor section 280 is intermittently performed by being controlled in the processing section 240. Therefore, a power supply which is supplied to the sensor section 280 or the processing section 240 is intermittently consumed. Therefore, it is possible to realize the measurement device 1 in which it is possible to increase measurable time by suppressing power consumption and consumption of the battery 262.

## Claims

1. A biological information measurement device comprising:
a sensor section which detects biological information of a test object;
a processing section which performs processing of the biological information;
a power supply section which supplies electric power to the sensor section and the processing section;
a device main body having a case section in which the sensor section, the processing section, and the power supply section are accommodated; and
a band for mounting the device main body on the test object,
wherein weight of the device main body is less than or equal to 60 g, and it is possible to measure biological information continuously for 10 hours or more.

2. The biological information measurement device according to Claim 1, wherein detection of the biological information by the sensor section is intermittently performed.

3. The biological information measurement device according to Claim 1 or Claim 2, wherein a time interval of performing detection is changed based on the detected biological information.

4. The biological information measurement device according to any of Claims 1 to 3, wherein the biological information measurement device stores biological information of the test object at rest, and
when a change in the detected biological information is small compared to the biological information at rest, the time interval is extended, and when a change in the detected biological information is large, the time interval is shortened.

5. The biological information measurement device according to any of Claims 1 to 4, wherein the power supply section is provided with a rechargeable battery, and
the amount of charge of the battery is determined according to the number of times of detection or measurement time of the biological information performed after previous charging.

6. The biological information measurement device according to Claim 5, wherein the amount of charge of the battery is set in advance according to a desired number of times of detection or measurement time and the charging of the battery is performed.

7. The biological information measurement device according to Claim 5 or Claim 6, wherein capacity of the battery to be installed is selected according to the number of times of the detection or the measurement time which is performed after the charging.

8. The biological information measurement device according to any of Claims 1 to 6, wherein the time interval of performing the detection is changed according to the amount of charge of the battery.

9. A biological information measurement device comprising:
a sensor section which detects biological information of a test object;
a processing section which performs processing of the biological information;
a power supply section which supplies electric power to the sensor section and the processing section;
a device main body having a case section in which the sensor section, the processing section, and the power supply section are accommodated; and
a band for mounting the device main body on the test object,
wherein volume of the device main body of the biological information measurement device is less than or equal to 50 cc, and it is possible to measure biological information continuously for 10 hours or more.

10. The biological information measurement device according to Claim 9, wherein detection of the biological information by the sensor section is intermittently performed.

11. The biological information measurement device according to Claim 9 or Claim 10, wherein a time interval of performing detection is changed based on the detected biological information.

12. The biological information measurement device according to any of Claims 9 to 11, wherein the biological information measurement device stores biological information of the test object at rest, and
when a change in the detected biological information is small compared to the biological information at rest, the time interval is extended, and when a change in the detected biological information is large, the time interval is shortened.

13. A biological information measurement device comprising:
a sensor section which detects biological information of a test object;
a processing section which performs processing of the biological information;
a power supply section which supplies electric power to the sensor section and the processing section;
a device main body having a case section in which the sensor section, the processing section, and the power supply section are accommodated; and
a band for mounting the device main body on the test object,
wherein the maximum thickness of the device main body of the biological information measurement device is less than or equal to 16 mm, and it is possible to measure biological information continuously for 10 hours or more.

14. The biological information measurement device according to Claim 13, wherein detection of the biological information by the sensor section is intermittently performed.

15. The biological information measurement device according to Claim 13 or Claim 14, wherein a time interval of performing detection is changed based on the detected biological information.

16. The biological information measurement device according to any of Claims 13 to 15, wherein the biological information measurement device stores biological information of the test object at rest, and
when a change in the detected biological information is small compared to the biological information at rest, the time interval is extended, and when a change in the detected biological information is large, the time interval is shortened.
